(19) Europäisches Patentamt
European Patent Office
Office européen des brevets

(11) **EP 4 049 710 B1**

(12) **EUROPEAN PATENT SPECIFICATION**

(45) Date of publication and mention
of the grant of the patent:
**20.09.2023 Bulletin 2023/38**

(21) Application number: **22159021.9**

(22) Date of filing: **25.02.2022**

(51) International Patent Classification (IPC):
**A61N 1/04** *(2006.01)*    **A61N 1/32** *(2006.01)*
**A61N 1/378** *(2006.01)*

(52) Cooperative Patent Classification (CPC):
**A61N 1/0412; A61N 1/327;** A61N 1/378

(54) **HIGH VOLTAGE PULSE GENERATOR FOR ELECTRO-PORATION**

HOCHSPANNUNGSIMPULSGENERATOR ZUR ELEKTROPORATION

GÉNÉRATEUR D'IMPULSIONS HAUTE TENSION POUR ÉLECTROPORATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.02.2021 IT 202100004601**

(43) Date of publication of application:
**31.08.2022 Bulletin 2022/35**

(73) Proprietor: **IGEA S.p.A.**
**41012 Carpi (MO) (IT)**

(72) Inventors:
• **CADOSSI, Ruggero**
**41012 CARPI (MO) (IT)**

• **MARAZZI, Donata**
**41012 CARPI (MO) (IT)**
• **BERTACCHINI, Claudio**
**41012 CARPI (MO) (IT)**

(74) Representative: **Studio Torta S.p.A.**
**Via Viotti, 9**
**10121 Torino (IT)**

(56) References cited:
**WO-A1-00/42914     WO-A1-2017/117251**
**US-A- 3 958 577**

**Description**

TECHNICAL FIELD

The present invention relates to a high voltage pulse generator for electro-poration.

BACKGROUND ART

As it is known, electro-poration treatments involve the application of high voltage electrical pulses (about 1000 Volts) produced by a pulse generator to an organic tissue by means of the use of electrodes applied to the tissue itself; the electric field generated in the tissue produces the formation of pores in the cell plasma membrane causing a change in its permeability which facilitates the flow of organic/inorganic substances (such as DNA or medicinal products) from the outside to the inside of the cell. These electro-poration treatments are controllable based on the parameters (voltage, waveform, duty-cycle, application time, number of pulses applied, etc.) of the electrical pulses. In some applications the electrodes are inserted into the tissue to make the electro-poration process more effective; for example, the electrodes can comprise a plurality of needles that are electrically connected with the pulse generator and that are inserted inside the portion of the body of a patient to which the electro-poration treatment must be applied.

Pulse generators for electro-poration of the known type comprise a capacitor bank in which the electric charge used to generate the pulses is stored and an electronic switch which is switched by a driving signal to produce a given waveform of the pulses, for example a square wave.

The pulse generators of the known type, however, produce a waveform that is strongly dependent on the impedance of the organic tissue and for this reason the amplitude of the pulses in many operating conditions do not correspond to that expected, especially if the current absorbed by the tissue noticeably varies during the electro-poration treatment following the variation of the impedance, in particular following the decrease of the impedance. Document WO 2017/117251 discloses an electro-poration device.

DISCLOSURE OF INVENTION

The object of the present invention is to manufacture a pulse generator that allows good control of the pulse voltage even if the current absorbed by the load varies significantly following the variation of the impedance during treatment.

The preceding object is achieved by the present invention since it relates to a high voltage pulse generator for electro-poration comprising: a capacitor bank in which the electric charge used to generate the pulses is stored; the capacitor bank has an output terminal on which a high voltage direct current HT is present; a voltage amplifier circuit which receives, at input, the high voltage direct current HT and is provided with an automatic gain control circuit able to compare a reference voltage $V_{HT\text{-}ref}$ with the voltage supplied at output $\mathbf{V_{OUT}}$ by the amplifier to keep the output voltage constant during the generation of the pulses; a current amplifier circuit arranged downstream of the voltage amplifier circuit and provided with an electronic semiconductor switch communicating with a first terminal with the output of the capacitor bank and provided with a second terminal designed to communicate with an output of the pulse generator; the voltage present at the output of the voltage amplifier is supplied to the pulse generator output and is used to switch-off said electronic semiconductor switch.

BRIEF DESCRIPTION OF THE DRAWINGS

The invention will now be illustrated with reference to the attached drawings which represent a preferred non-limiting embodiment thereof wherein:

Figure 1 illustrates an electrical diagram of a high voltage pulse generator for electro-poration made according to the present invention; and
Figure 2 illustrates pulses obtained from a known type of pulse generator; and
Figure 3 illustrates experimental data illustrating the waveform produced by the pulse generator according to the present invention.

BEST MODE FOR CARRYING OUT THE INVENTION

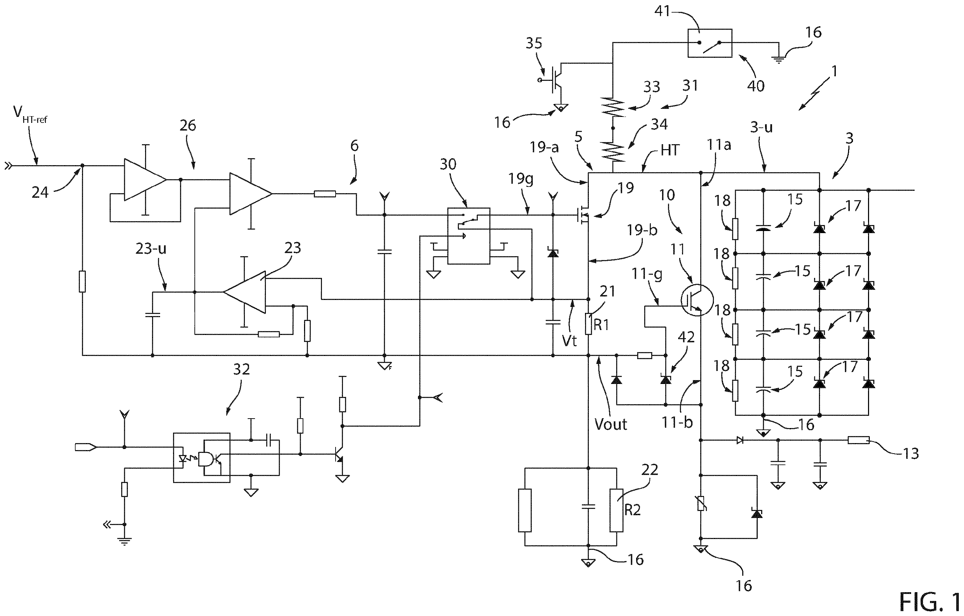

**In Figure 1, 1 denotes,** as a whole, a high voltage pulse generator for electro-poration. The terms high voltage pulses refer to pulses having a voltage of the order of about 1000 Volts (in some applications the pulses can reach values of 3000 V).

The pulse generator 1 comprises:
a capacitor bank 3 in which the electric charge used to generate the pulses is stored; the capacitor bank 3 has an output terminal 3u on which a high voltage direct current HT is present (for example 1200 Volt).

A voltage amplifier circuit 5 which receives, at input, the high voltage direct current HT and is provided with an automatic gain control circuit 6 designed to compare a reference voltage $V_{HT\text{-}ref}$ with an output voltage Vr from the amplifier 5 to keep the output voltage $V_{OUT}$ constant during the generation of the pulses;

a current amplifier circuit 10 arranged downstream of the voltage amplifier circuit 5 and provided with an electronic semiconductor switch 11 communicating with its first terminal 11-a with the output terminal 3u of the capacitor bank 3 and provided with a second terminal 11-b designed to communicate with an output 13 of the pulse generator 1;
the voltage $V_{OUT}$ present at the output of the voltage amplifier 5 is supplied to the output of the pulse generator (less than a small potential drop $\Delta Z$ of a few volts) and is used to switch-off the electronic semiconductor switch 11.

An electrode for electro-poration (of a known type and not shown) can be connected to the output 13 and applied to a patient which represents a load for the pulse generator 1. a) The voltage applied to the tissue in clinical practice is always expressed as V/cm.

Preferably the capacitor bank 3 is formed by a plurality of electrolytic capacitors 15 arranged in series with one another between a reference terminal 16 (ground) and the output terminal 3u. For example, four 1000 $\mu$F 400Volt capacitors can be used. Each capacitor 15 is provided with a respective protection and charge balancing circuit formed respectively by a Zener diode 17 (or by a pair of Zener diodes arranged in parallel as illustrated) and by a resistor 18 arranged in parallel with the capacitor 15. A typical resistance of resistor 18 value is 10 MOhm. A non-limiting example of the Zener diode 17 is the p6ke440a. The charging voltage of the capacitor bank 3 can be detected by means of a resistive divider (not illustrated) and an AD converter designed to measure the voltage present on the resistive divider. In turn, the capacitors 15 are charged by a voltage boosting circuit of a known type (not illustrated).

The voltage amplifier circuit 5 comprises a second electronic switch 19 (in the example a power MOSFET) having a first terminal 19-a communicating with the output terminal 3u of the capacitor bank 3 and a second terminal 19-b connected with the reference terminal 16 (ground) via a first resistor 21 (resistance value R1) and a second resistor 22 (resistance value R2 much higher than R1 for example R1 = 10 Ohm R2 = 100KOhm) making a voltage divider; the automatic gain control circuit 6 is configured to compare the voltage Vr present on the first resistor 21 with the reference voltage $V_{HT-ref}$ to produce a driving signal of the second electronic switch 19, in the example the signal supplied to the gate 19g of the MOSFET. The voltage Vr is in fact proportional to the voltage Vout according to:

$$Vr= Vout - R2*HT/(R1+R2)$$

The automatic gain control circuit 6 comprises a non-inverting amplifier 23 which receives, at input, the voltage Vr at the ends of the first resistor 21 and that has an output 23-u in which a voltage is present, which is compared with the reference voltage $V_{HT-ref}$ in an adder node 24.

The reference voltage $V_{HT-ref}$ has a periodic trend (for example square wave) which regulates the waveform and the frequency of the pulse generator signal 1.

The difference between $V_{HT-ref}$ and the output voltage of the non-inverting amplifier 23 is supplied to the input of a follower amplifier 26. The output of the follower amplifier 26 produces the driving signal of the second electronic switch 19.

A circuit breaker switch 30 is arranged between the output of the follower amplifier 26 and the control terminal (gate 19g) of the second electronic semiconductor switch. A control circuit 32 is configured to achieve the opening of the circuit breaker switch 30 when, for any reason, one wishes to turn off the switch 19 and consequently also the switch 10, thereafter interrupting the supply of the driving signal to the second electronic switch 19 that opens instantly.

The pulse generator 1 comprises a controlled discharge circuit 31 of the capacitors 15, so as to reduce the voltage on the capacitors to a desired value without necessarily having to completely discharge the capacitors 15 and then recharge the same, and which connects the output terminal 3u with the ground 16 through of a pair of dissipator resistors 33, 34 connected in series. Said circuit can also be used so as to prevent the risks associated with the high voltage present in the capacitors 15. The activation of the controlled discharge circuit 31 is achieved by sending a closing signal to a third electronic semiconductor switch 35 (for example an IGBT) arranged between the resistor 33 and the ground 16. The third electronic semiconductor switch 35 is open during the operation of the pulse generator 1.

The pulse generator 1 comprises a quick discharge circuit 40 of the capacitors 15 which connects the output terminal 3u with the ground 16 through the resistors 33 and 34 through a quick-closing relay 41. The use of a relay 41 achieves a quick and deep discharge in case of emergency. In fact, it has been seen that the discharge IGBT 35 is unable to discharge resistors 33 and 34 to zero but leaves a residual voltage of a couple of tens of volts.

The electronic semiconductor switch 11 cooperates with a Zener diode 42 interposed between the control terminal 11g (in the example the gate of the IGBT 11) and the second terminal of the electronic switch (emitter of the IGBT) to obtain the appropriate gate voltage. The voltage Vout is applied both to the control terminal 11g and to the cathode of the Zener diode 42. In this way, in the presence of a Vout, the voltage applied between the gate and the emitter of the IGBT 11 corresponds to the voltage of the Zener (for example 9 volts) and is higher than the threshold value of the IGBT which can work in saturation by letting flow all the current required by the load and coming from the capacitors 15. The voltage across the Zener 42 produces the potential drop $\Delta Z$.

The advantages of the pulse generator 1 are as follows:

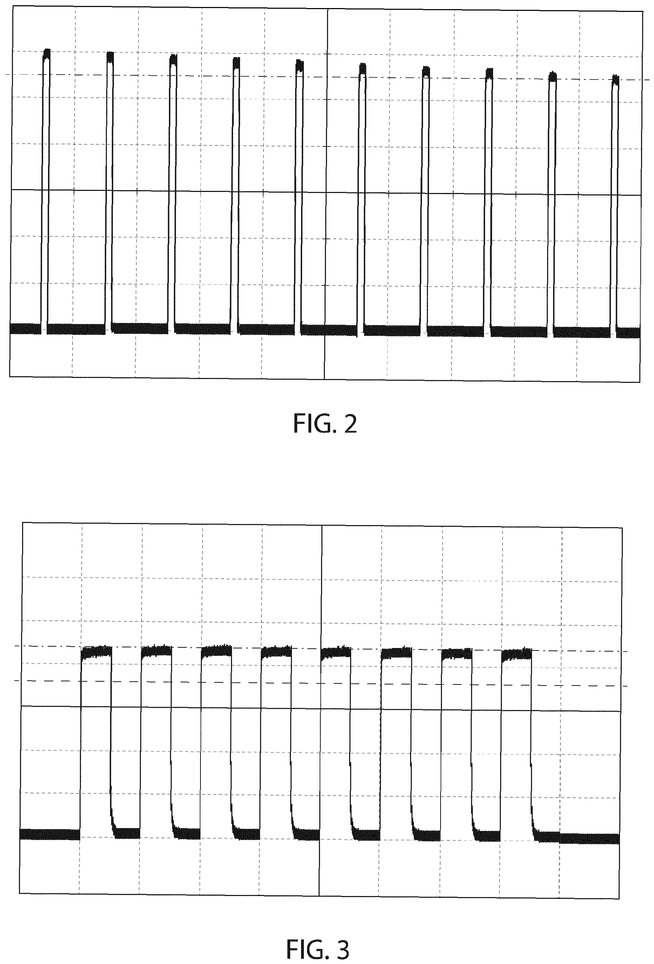

- obtain pulses with stable amplitude during generation of the same.
- Being able to obtain pulses with an arbitrary shape;
- the mode of generation of the pulses allows to keep the single pulses largely homogeneous; consider Figure 2 which illustrates pulses obtained with known technologies and Figure 3 which illustrates pulses obtained by the pulse generator according to the present invention;

- emphasize that the repeatability and homogeneity of the pulses is important to ensure effective electro-poration of all cells in the tissue.

## Claims

1.  A high voltage pulse generator for electro-poration comprising

    - a capacitor bank (3) in which the electric charge used to generate the pulses is stored; the capacitor bank (3) has an output terminal (3u) on which a high voltage direct current HT is present;
    - **characterized by** comprising a voltage amplifier circuit (5) which receives, at input, the high voltage direct current HT and is provided with an automatic gain control circuit (6) designed to compare a reference voltage $V_{HT-ref}$ with the voltage supplied at output $V_{OUT}$ by the amplifier (5) to maintain the voltage constant at output during generation of the pulses;
    - a current amplifier circuit (10) arranged downstream of the voltage amplifier circuit (5) and provided with an electronic semiconductor switch (11) communicating with a first terminal (11-a) with the output of the capacitor bank (3) and provided with a second terminal (11a) designed to communicate with an output (13) of the pulse generator;
    - the voltage present at the output of the voltage amplifier (5) is supplied to the output (13) of the pulse generator and is used to operate the switching during closing of said electronic semiconductor switch (11).

2.  The pulse generator according to claim 1 wherein the capacitor bank (3) is formed by a plurality of electrolytic capacitors (15) arranged in series with one another between a reference terminal (16, ground) and the output terminal (3u) of the capacitor bank (3); each capacitor (15) is provided with a respective protection and charge balancing circuit formed respectively by a Zener diode (17) and by a resistor (18) arranged in parallel to the capacitor (15).

3.  The generator according to claim 1 or 2, wherein the voltage amplifier circuit (5) comprises a second electronic semiconductor switch (19) having a first terminal (19-a) communicating with the output terminal (3u) of the capacitor bank (3) and a second terminal (19-b) connected to a reference terminal (16, ground) via a first resistor (21, R1) and a second resistor (22, R2) forming a voltage divider; the automatic gain control circuit (6) is configured to compare the voltage Vr present on the first resistor (21) with the reference voltage $V_{HT-ref}$ to produce a driving signal of the second electronic switch (19).

4.  The pulse generator according to claim 3, wherein the automatic gain control circuit (6) comprises a non-inverting amplifier (23) which receives at input the voltage Vr at the ends of the first resistor (21) and which has an output (23-u) which is compared with the reference voltage $V_{HT-ref}$; the difference between the reference voltage $V_{HT-ref}$ and the output voltage of the non-inverting amplifier (23) is supplied to the input of a follower amplifier (26); the output of the follower amplifier (26) produces the driving signal of the second electronic switch (19).

5.  The pulse generator according to claim 4, wherein a circuit breaker switch (30) is arranged between the output of the comparator amplifier (26) and the control terminal (gate 19g) of the second electronic semiconductor switch (19); a control circuit (32) is configured to achieve the opening of the circuit breaker switch (30) when a potentially dangerous situation is detected, thereafter interrupting the supply of the driving signal to the second electronic switch (19) which opens instantaneously.

6.  The pulse generator according to claim 2, wherein a controlled discharge circuit (31) of the capacitors (15) is provided, which is configured to arrange in connection the output terminal (3u) of the capacitor bank with the reference terminal (ground 16) through a dissipator resistor (33, 34) and a semiconductor switch (35) after the switching-off of the pulse generator (1).

7.  The pulse generator according to claim 1, wherein a quick discharge circuit (40) of the capacitors (15) is provided, which is configured to arrange in connection the output terminal (3u) of the capacitor bank with the reference terminal (ground 16) through a dissipator resistor (33, 34) and a quick-closing relay (41) after the switching-off of the pulse generator (1).

8.  The pulse generator according to one of the preceding claims, wherein the electronic semiconductor switch (11) cooperates with a Zener diode (42) interposed between a control terminal (11g) and a reference terminal of the electronic switch (11); the voltage supplied at output $V_{OUT}$ is furthermore applied to the cathode of the Zener diode (42) which has a voltage higher than the threshold voltage of the electronic switch (11) which can work in saturation leaving the current coming from the capacitor bank (15) towards a load of the pulse generator.

**Patentansprüche**

1. Hochspannungsimpulsgenerator für Elektroporation, der aufweist

   - eine Kondensatorbank (3), in der die zur Erzeugung des Impulses verwendete elektrische Ladung gespeichert ist; wobei die Kondensatorbank (3) einen Ausgangspol (3u) aufweist, an dem ein Hochspannungs-Gleichstrom HT anliegt;
   - **dadurch gekennzeichnet, dass** die Vorrichtung eine Spannungsverstärkerschaltung (5) aufweist, die am Eingang den Hochspannungs-Gleichstrom HT empfängt und mit einer Schaltung zur automatischen Verstärkungsregelung ausgestattet ist, die so ausgestaltet ist, dass sie eine Referenzspannung $V_{HT-ref}$ mit der vom Verstärker (5) am Ausgang $V_{OUT}$ zugeführten Spannung vergleicht, um die Spannung am Ausgang während der Erzeugung des Impulses konstant beizubehalten;
   - eine Stromverstärkerschaltung (10), die stromabwärts der Spannungsverstärkerschaltung (5) angeordnet und mit einem elektronischen Halbleiterschalter (11) ausgestattet ist, der mit einem ersten Pol (11-a) mit dem Ausgang der Kondensatorbank (3) kommuniziert und mit einem zweite Pol (11a) ausgestattet ist, der so ausgestaltet ist, dass er mit einem Ausgang (13) des Impulsgenerators kommuniziert;
   - die am Ausgang des Spannungsverstärkers (5) angelegte Spannung wird dem Ausgang (13) des Impulsgenerators zugeführt und dazu verwendet, die Schaltung beim Schließen des elektronischen Halbleiterschalters (11) zu betätigen.

2. Impulsgenerator nach Anspruch 1, wobei die Kondensatorbank (3) durch eine Vielzahl von Elektrolytkondensatoren (15) gebildet wird, die in Reihe zueinander zwischen einem Referenzpol (16, Masse) und dem Ausgangspol (3u) der Kondensatorbank (3) angeordnet sind; wobei jeder Kondensator (15) mit einer entsprechenden Schutz- und Ladungsausgleichsschaltung ausgestattet ist, die jeweils durch einer Zener-Diode (17) und durch einen Widerstand (18) gebildet wird, die parallel zu dem Kondensator (15) angeordnet sind.

3. Generator nach Anspruch 1 oder 2, wobei die Spannungsverstärkerschaltung (5) einen zweiten elektronischen Halbleiterschalter (19) aufweist mit einem ersten Pol (19-a), der mit dem Ausgangspol (3u) der Kondensatorbank (3) kommuniziert, und einem zweiten Pol (19-b), der mit einem Referenzpol (16, Masse) über einen ersten Widerstand (21, R1) und einen zweiten Widerstand (22, R2), die einen Spannungsteiler bilden, verbunden ist; wobei die Schaltung zur automatischen Verstärkungsregelung (6) dazu ausgebildet ist, die an dem ersten Widerstand (21) angelegte Spannung Vr mit der Referenzspannung $V_{HT-ref}$ zu vergleichen, um ein Antriebssignal der zweiten elektronischen Schaltung (19) zu erzeugen.

4. Impulsgenerator nach Anspruch 3, wobei die Schaltung zur automatischen Verstärkungsregelung (6) einen nichtinvertierenden Verstärker (23) aufweist, der am Eingang die Spannung Vr an den Enden des ersten Widerstands (21) empfängt und einen Ausgang (23-u) aufweist, der mit der Referenzspannung $V_{HT-ref}$ verglichen wird; wobei die Differenz zwischen der Referenzspannung $V_{HT-ref}$ und der Ausgangsspannung des nichtinvertierenden Verstärkers (23) dem Eingang eines Folgeverstärkers (26) zugeführt wird; wobei der Ausgang des Folgeverstärkers (26) das Antriebssignal der zweiten elektronischen Schaltung (19) erzeugt.

5. Impulsgenerator nach Anspruch 4, wobei ein Leistungsschalter (30) zwischen dem Ausgang des Komparatorverstärkers (26) und dem Steuerpol (Gate 19g) des zweiten elektronischen Halbleiterschalters (19) angeordnet ist; wobei ein Regelkreis (32) dazu ausgebildet ist, das Öffnen des Leistungsschalters (30) zu erzielen, wenn eine potenziell gefährliche Situation erkannt wird, wonach die Zufuhr des Antriebssignals an den zweiten elektronischen Schalter (19), der sich sofort öffnet, unterbrochen wird.

6. Impulsgenerator nach Anspruch 2, wobei eine gesteuerte Entladeschaltung (31) der Kondensatoren (15) vorgesehen ist, die dazu ausgebildet ist, den Ausgangspol (3u) der Kondensatorbank mit dem Referenzpol (Masse 16) über einen Ableitwiderstand (33, 34) und einen Halbleiterschalter (35) nach dem Ausschalten des Impulsgenerators (1) in Verbindung bringen.

7. Impulsgenerator nach Anspruch 1, wobei eine Schnellentladeschaltung (40) der Kondensatoren (15) vorgesehen ist, die dazu ausgebildet ist, den Ausgangspol (3u) der Kondensatorbank mit dem Referenzpol (Masse 16) über einen Ableitwiderstand (33, 34) und ein Schnellschlussrelais (41) nach dem Ausschalten des Impulsgenerators (1) in Verbindung bringen.

8. Impulsgenerator nach einem der vorstehenden Ansprüche, wobei der elektronische Halbleiterschalter (11) mit einer zwischen einem Steuerpol (11g) und einem Referenzpol des elektronischen Schalters (11) angeordneten Zener-Diode (42) zusammenwirkt;
   die am Ausgang $V_{OUT}$ zugeführte Spannung ferner an die Kathode der Zener-Diode (42) angelegt wird, die eine höhere Spannung als die Schwellenwertspannung des elektronischen Schalters (11) aufweist, der in Sättigung arbeiten kann, so dass der von der Kondensatorbank (15) kommende Strom zu einer Last des Impulsgebers fließt.

**Revendications**

1. Générateur d'impulsions à haute tension pour électroporation comprenant

    - une batterie de condensateurs (3) dans laquelle la charge électrique utilisée pour générer les impulsions est stockée ; la batterie de condensateurs (3) possède une borne de sortie (3u) sur laquelle un courant continu à haute tension HT est présent ;
    - **caractérisé par le fait qu'**il comprend un circuit amplificateur de tension (5) qui reçoit, en entrée, le courant continu à haute tension HT et est muni d'un circuit de commande de gain automatique (6) conçu pour comparer une tension de référence $V_{HT-ref}$ avec la tension alimentée en sortie $V_{OUT}$ par l'amplificateur (5) pour maintenir la tension constante en sortie pendant la génération des impulsions ;
    - un circuit amplificateur de courant (10) agencé en aval du circuit amplificateur de tension (5) et muni d'un commutateur à semi-conducteurs électronique (11) communiquant avec une première borne (11-a) avec la sortie de la batterie de condensateurs (3) et muni d'une seconde borne (11a) conçue pour communiquer avec une sortie (13) du générateur d'impulsions ;
    - la tension présente à la sortie de l'amplificateur de tension (5) alimente la sortie (13) du générateur d'impulsions et est utilisée pour mettre en oeuvre la commutation pendant la fermeture dudit commutateur à semi-conducteurs électronique (11).

2. Générateur d'impulsions selon la revendication 1 dans lequel la batterie de condensateurs (3) est formée par une pluralité de condensateurs électrolytiques (15) agencés en série les uns avec les autres entre une borne de référence (16, masse) et la borne de sortie (3u) de la batterie de condensateurs (3) ; chaque condensateur (15) est muni d'un circuit de protection et d'équilibrage de charge respectif formé respectivement par une diode Zener (17) et par une résistance (18) agencées parallèlement au condensateur (15).

3. Générateur selon la revendication 1 ou 2, dans lequel le circuit amplificateur de tension (5) comprend un second commutateur à semi-conducteurs électronique (19) ayant une première borne (19-a) communiquant avec la borne de sortie (3u) de la batterie de condensateurs (3) et une seconde borne (19-b) connectée à une borne de référence (16, masse) via une première résistance (21, R1) et une seconde résistance (22, R2) formant un diviseur de tension ; le circuit de commande de gain automatique (6) est configuré pour comparer la tension Vr présente sur la première résistance (21) avec la tension de référence $V_{HT-ref}$ pour produire un signal d'attaque du second commutateur électronique (19).

4. Générateur d'impulsions selon la revendication 3, dans lequel le circuit de commande de gain automatique (6) comprend un amplificateur non inverseur (23) qui reçoit en entrée la tension Vr aux bornes de la première résistance (21) et qui possède une sortie (23-u) qui est comparée avec la tension de référence $V_{HT-ref}$ ; la différence entre la tension de référence $V_{HT-ref}$ et la tension de sortie de l'amplificateur non inverseur (23) alimente l'entrée d'un amplificateur suiveur (26) ; la sortie de l'amplificateur suiveur (26) produit le signal d'attaque du second commutateur électronique (19).

5. Générateur d'impulsions selon la revendication 4, dans lequel un commutateur disjoncteur (30) est agencé entre la sortie de l'amplificateur comparateur (26) et la borne de commande (grille 19g) du second commutateur à semi-conducteurs électronique (19) ; un circuit de commande (32) est configuré pour accomplir l'ouverture du commutateur disjoncteur (30) lorsqu'une situation potentiellement dangereuse est détectée, ce qui interrompt par la suite l'alimentation du signal d'attaque au second commutateur électronique (19) qui s'ouvre instantanément.

6. Générateur d'impulsions selon la revendication 2, dans lequel un circuit de décharge commandée (31) des condensateurs (15) est ménagé, qui est configuré pour agencer en connexion la borne de sortie (3u) de la batterie de condensateurs avec la borne de référence (masse 16) par le biais d'une résistance à dissipateur (33, 34) et d'un commutateur à semi-conducteurs (35) après la coupure du générateur d'impulsions (1).

7. Générateur d'impulsions selon la revendication 1, dans lequel un circuit de décharge rapide (40) des condensateurs (15) est ménagé, qui est configuré pour agencer en connexion la borne de sortie (3u) de la batterie de condensateurs avec la borne de référence (masse 16) par le biais d'une résistance à dissipateur (33, 34) et d'un relais à fermeture rapide (41) après la coupure du générateur d'impulsions (1).

8. Générateur d'impulsions selon l'une des revendications précédentes, dans lequel le commutateur à semi-conducteurs électronique (11) coopère avec une diode Zener (42) interposée entre une borne de commande (11g) et une borne de référence du commutateur électronique (11) ;

la tension alimentée en sortie $V_{OUT}$ est en outre appliquée à la cathode de la diode Zener (42) qui possède une tension plus élevée que la tension de seuil du commutateur électronique (11) qui peut travailler en saturation en laissant le courant aller de la batterie de condensateurs (15) vers une charge du générateur d'impulsions.

FIG.1

FIG. 2

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017117251 A